# EUROPÄISCHE PATENTANMELDUNG

(11) **EP 0 725 063 A1**
(43) Veröffentlichungstag der Anmeldung: **07.08.1996**
(21) Anmeldenummer: 95810066.1
(22) Anmeldetag: 01.02.1995
(51) Int. Cl.: C07D 215/18, C07D 215/04, C07D 215/12, C07D 401/12, A61K 31/47

(54) **Chinolin-Derivate**

(71) Anmelder: CIBA-GEIGY AG, 4002 Basel (CH)
(72) Erfinder: von Sprecher, Andreas, Dr., CH-4104 Oberwil (CH); Beck, Andreas, Dr., D-79108 Freiburg (DE); Gerspacher, Marc, Dr., CH-5200 Brugg (CH)

(57) **Zusammenfassung**

Verbindungen der Formel I 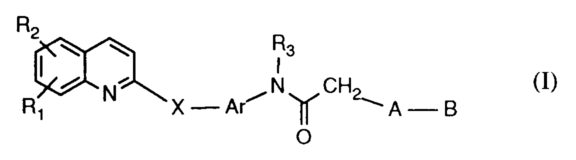
worin R₁-R₃, X, Ar, A und B die in der Beschreibung angegebene Bedeutung haben, weisen wertvolle pharmazeutische Eigenschaften auf und sind insbesondere als Leukotrien-Antagonisten wirksam. Sie werden in an sich bekannter Weise hergestellt.

## Beschreibung

Die Erfindung betrifft Verbindungen der Formel I, 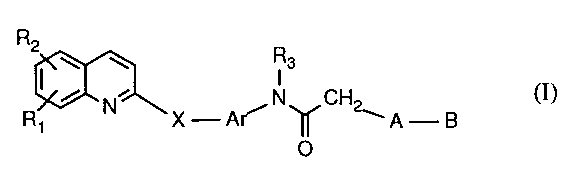
worin
R₁ und R₂ unabhängig voneinander Wasserstoff, Niederalkyl, Halogen-niederalkyl, Arylniederalkyl, Cycloalkyl, Halogen, Hydroxy, Niederalkoxy, Halogen-niederalkoxy, Arylniederalkoxy, Acyloxy, Mercapto, Niederalkyl(-thio, -sulfinyl oder -sulfonyl), Amino, Niederalkylamino, Diniederalkylamino, Acylamino, Nitro, Acyl, Carboxy, Niederdkoxycarbonyl, Aminocarbonyl, N-Niederalkylaminocarbonyl, N,N-Diniederalkylaminocarbonyl oder Cyano bedeuten, oder
R₁ und R₂ zusammen -(CH₂)ₘ-, worin m für 3, 4 oder 5 steht, bedeuten,
X für eine Gruppe -CH₂-O- (wobei CH₂ mit dem Chinolinrest und O mit der Gruppe Ar verknüpft ist) oder eine Gruppe -CH=CH- steht,
Ar für 1,3-Phenylen oder 2,7-Naphthylen steht,
R₃ Wasserstoff oder Niederalkyl bedeutet,
A für eine Gruppe -C(R₄R₅)- oder eine Gruppe 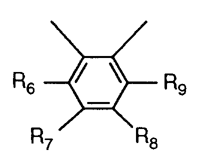
steht, wobei
R₄ und R₅ unabhängig voneinander Niederalkyl bedeuten, oder
R₄ und R₅ zusammen -(CH₂)ₙ-, worin n für 3, 4, 5 oder 6 steht, bedeuten, und
R₆, R₇, R₈ und R₉ unabhängig voneinander für Wasserstoff, Niederalkyl,
Halogen-niederalkyl, Halogen, Hydroxy, Niederalkoxy oder Niederalkylthio stehen, oder
R₇ und R₈ zusammen einen Benzoring bilden, und
B 5-Tetrazolyl oder eine Gruppe -C(=O)-Y bedeutet wobei
Y Hydroxy, Niederalkoxy, Amino, Niederalkylamino oder Diniederalkylamino bedeutet;
mit der Massgabe, dass A von einer Gruppe -C(R₄R₅)- verschieden ist, wenn B eine Gruppe -C(=O)-Y bedeutet,
und Salze davon, Verfahren zur Herstellung dieser Verbindungen, pharmazeutische Präparate, die diese Verbindungen enthalten, die Verwendung dieser Verbindungen zur therapeutischen Behandlung des menschlichen oder tierischen Körpers oder zur Herstellung pharmazeutischer Präparate.

Die vor- und nachstehend verwendeten Allgemeinbegriffe haben im Rahmen der vorliegenden Anmeldung vorzugsweise die folgenden Bedeutungen:

Das Präfix "Nieder" bezeichnet einen Rest bis und mit 7 und insbesondere bis und mit 4 Kohlenstoffatomen.

Niederalkyl ist z.B. n-Propyl, Isopropyl, n-Butyl, Isobutyl, sek-Butyl, tert-Butyl, n-Pentyl, Neopentyl, n-Hexyl oder n-Heptyl, vorzugsweise Ethyl und vor allem Methyl.

C₁-C₃-Alkyl steht für Methyl, Ethyl, n-Propyl und Isopropyl.

Niederalkyl als Bedeutung von R₄ und R₅ ist bevorzugt Ethyl.

Halogen-niederalkyl ist z.B. Trifluormethyl.

Halogen-niederalkoxy ist z.B. Trifluormethoxy.

Cycloalkyl ist vorzugsweise C₃-C₈- und insbesondere C₅-C₆-Cycloalkyl, was bedeuten soll, dass es 3 bis 8 bzw. 5 bis 6 Ringkohlenstoffe enthält. Cycloalkyl kann aber auch substituiert sein, z.B. durch Niederalkyl.

Halogen steht insbesondere für Chlor oder Brom, vor allem für Fluor, kann aber auch Iod bedeuten.

Acyl steht z.B. für Niederalkanoyl; Niederalkanoyl ist z.B. Acetyl, Propionyl oder Pivaloyl, aber etwa auch Formyl.

Aminocarbonyl ist die Gruppe -CONH₂.

Stehen R₁ und R₂ zusammen für den bivalenten Rest -(CH₂)ₘ-, so ist dieser bivalente Rest vorzugsweise an zwei benachbarten C-Atomen des Phenylrings angeknüpft (z.B. als 6,7-Cyclopentanochinolinyl).

Bedeuten in einer Gruppe -C(R₄R₅)- die Reste R₄ und R₅ beide Ethyl, so wird das im folgenden als -C(C₂H₅)₂- oder als "Diethylmethylen" ausgedrückt.

Stehen R₄ und R₅ zusammen für den bivalenten Rest -(CH₂)ₙ-, so ist dieser bivalente Rest zweimal am selben C-Atom angeknüpft, so dass ein Cycloalkan gebildet wird.

Bedeuten die Reste R₆-R₉ in einer Gruppe 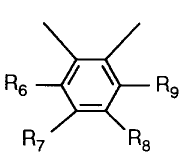
alle Wasserstoff, so entspricht die Gruppe dem Rest 1,2-Phenylen. Bilden R₇ und R₈ zusammen einen Benzoring, so bedeutet die Gruppe 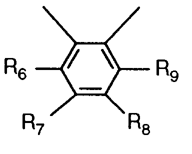
ein gegebenenfalls substituiertes 2,3-Naphthylen.

Aryl ist z.B. Phenyl, das unsubstituiert oder substituiert ist. Aryl ist bevorzugt Phenyl, das unsubstituiert oder durch einen oder mehrere, insbesondere einen oder zwei, Substituenten aus der Gruppe bestehend aus Niederalkyl, Niederalkoxy, Niederalkenyloxy, Hydroxy, Niederalkanoyloxy, Nitro, Amino, Halogen, Trifluormethyl, Carboxy, Niederalkoxycarbonyl, Aminocarbonyl, N-Niederalkylaminocarbonyl, N,N-Diniederalkylaminocarbonyl, Cyano, Niederalkanoyl, Benzoyl und Niederalkylsulfonyl substituiert ist. In erster Linie bedeutet Aryl Phenyl, das unsubstituiert oder durch Niederalkyl, Niederalkoxy, Niederalkenyloxy, Hydroxy, Halogen oder Trifluormethyl substituiert ist, und vor allen Dingen Phenyl.

1,3-Phenylen und 2,7-Naphthylen stehen für die bivalenten Reste 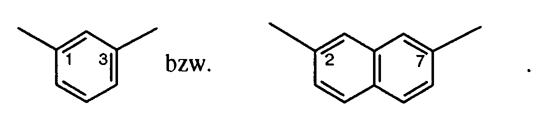
Salze von Verbindungen der Formel I sind insbesondere pharmazeutisch verwendbare Salze, vor allem Salze mit Basen, wie entsprechende Alkalimetall- oder Erdalkalimetallsalze, z.B. Natrium-, Kalium- oder Magnesiumsalze, pharmazeutisch verwendbare Uebergangsmetallsalze, wie Zink- oder Kupfersalze, oder Salze mit Ammoniak oder organischen Aminen, wie cyclischen Aminen, wie Mono-, Di- oder Triniederalkylaminen, wie Hydroxyniederalkylaminen, z.B. Mono-, Di- oder Trihydroxyniederalkylaminen, Hydroxyniederalkyl-niederalkyl-aminen oder Polyhydroxyniederalkylaminen. Cyclische Amine sind z.B. Morpholin, Thiomorpholin, Piperidin oder Pyrrolidin. Als Mononiederalkylamine kommen beispielsweise Ethyl- und t-Butylamin, als Diniederalkylamine beispielsweise Diethyl- und Diisopropylamin und als Triniederalkylamine beispielsweise Trimethyl- und Triethylamin in Betracht. Entsprechende Hydroxyniederalkylamine sind z.B. Mono-, Di- und Triethanolamin; Hydroxyniederalkyl-niederalkyl-amine sind z.B. N,N-Dimethylamino- und N,N-Diethylamino-ethanol; als Polyhydroxyniederalkylamin kommt z.B. Glucosamin in Frage. In bestimmten Fällen können auch Säureadditionssalze, beispielsweise mit starken anorganischen Säuren, wie Mineralsäuren, z.B. Schwefelsäure, einer Phosphorsäure oder einer Halogenwasserstoffsäure, mit starken organischen Carbonsäuren, wie Niederalkancarbonsäuren, z.B. Essigsäure, wie gegebenenfalls ungesättigten Dicarbonsäuren, z.B. Malon-, Malein- oder Fumarsäure, oder wie Hydroxycarbonsäuren, z.B. Wein- oder Zitronensäure, oder mit Sulfonsäuren, wie Niederalkan- oder gegebenenfalls substituierten Benzolsulfonsäuren, z.B. Methan- oder p-Toluolsulfonsäure, gebildet werden. Verbindungen der Formel I mit einer sauren Gruppe, z.B. Carboxy, und einer basischen Gruppe, z.B. Amino, können z.B. auch in Form von inneren Salzen, d.h. in zwitterionischer Form vorliegen, oder es kann ein Teil des Moleküls als inneres Salz, und ein anderer Teil als normales Salz vorliegen. Umfasst sind ferner für pharmazeutische Verwendungen ungeeignete Salze, da diese beispielsweise für die Isolierung bzw. Reinigung von freien Verbindungen I sowie von deren pharmazeutisch verwendbaren Salzen verwendet werden können.

Die Verbindungen der Formel I weisen wertvolle pharmakologische Eigenschaften, insbesondere eine ausgeprägte antagonistische Wirkung gegenüber Leukotrienen, auf.

So hemmen sie z.B. in vitro mit einem IC₅₀-Wert von etwa 0,1 bis etwa 50 nM die durch Leukotrien D₄ (LTD₄) induzierte Kontraktion des Meerschweinchen-Dünndarms. Diese als LTD₄-Antagonismus bezeichnete Wirkung kann experimentell z.B. verifiziert werden, indem an Meerschweinchen-Ileum-Segmenten in einem Organbad [Standardmethode: Tyrode-Lösung bei 38°C unter Begasung mit Oxicarbon (Mischung aus Sauerstoff und CO₂) unter Belastung von 1 g] mit synthetischem Leukotrien D₄ (in Form des Kaliumsalzes) Kontraktionen ausgelöst und diese isotonisch registriert werden. Das Ausmass der Hemmung der Kontraktionen durch die Prüfsubstanz wird nach einer Vorinkubation von 2 Minuten in Form der IC₅₀-Werte ermittelt, wobei als IC₅₀ diejenige Konzentration bezeichnet wird, bei welcher die Testkontraktionen um 50 % reduziert sind.

Die Verbindungen der Formel I sind auch in vivo ausgezeichnet wirksam. So kann z.B. im Bronchospasmus-Standardtest am Meerschweinchen nach oraler Verabreichung der Testsubstanz ein deutlicher antagonistischer Effekt gegenüber LTD₄ beobachtet werden. Die Verbindungen der Formel I zeichnen sich aus durch eine hohe Wirkungsaktivität und lange Wirkungsdauer. Zum Beispiel werden die Testsubstanzen den Tieren als Suspension in Methylcellulose mittels Magensonde verabreicht. Bei Behandlungszeiten bis zu 8 Stunden wird im Bereich von etwa 0,05 bis 2,0 mg/kg eine markante Reduktion der durch LTD₄ erzeugten Bronchokonstriktion beobachtet.

Exzellente Wirkungen zeigen die Verbindungen der Formel I auch im durch Leukotrien E₄ (LTE₄) induzierten Bronchospasmus, zum Beispiel bei oraler Verabreichung. Bei Behandlungszeiten bis zu 8 Stunden wird im Bereich von etwa 0,001 bis 1,0 mg/kg p.o. wiederum eine starke Wirksamkeit beobachtet.

Die Verbindungen der Formel I können daher z.B. überall dort therapeutische Anwendung finden, wo die Wirkung der Leukotriene zu krankhaften Zuständen führt, und diese mildern oder beheben. Die Leukotriene spielen eine wichtige Rolle u.a. bei der Entstehung von allergischen und entzündlichen Prozessen. Demzufolge können die Verbindungen der Formel I z.B. als Antiallergika verwendet werden, etwa zur Behandlung von allergischen Zuständen und Erkrankungen, wie insbesondere Asthma, aber auch z.B. bei Heufieber oder obstruktiven Lungenkrankheiten. Die Verbindungen der Formel I können z.B. auch zur Behandlung von entzündlichen Erkrankungen der Lunge und anderer Organe verwendet werden, z.B. bei zystischer Fibrose oder Schocklunge (Adult Respiratory Distress Syndrome), aber auch z.B. bei Psoriasis, Colitis ulcerosa oder Morbus Crohn, bei septischem Schock oder entzündlichen Erkrankungen des Auges.

Die Erfindung betrifft vorzugsweise die Verbindungen der Formel I, worin

R₁ und R₂ unabhängig voneinander Wasserstoff, Niederalkyl, Halogen-niederalkyl, Phenylniederalkyl, Halogen, Hydroxy, Niederalkoxy, Halogen-niederalkoxy, Phenylniederalkoxy, Niederalkyl-(thio, sulfinyl oder sulfonyl), Nitro, Niederalkanoyl oder Cyano bedeuten, oder
R₁ und R₂ zusammen -(CH₂)₃- oder -(CH₂)₄- bedeuten,
X für für eine Gruppe -CH₂-O- (wobei CH₂ mit dem Chinolinrest und O mit der Gruppe Ar verknüpft ist) oder eine Gruppe -CH=CH- steht,
Ar für 1,3-Phenylen oder 2,7-Naphthylen steht,
R₃ Wasserstoff oder Niederalkyl bedeutet,
A für eine Gruppe -C(R₄R₅)- oder eine Gruppe 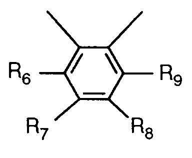
steht, wobei
R₄ und R₅ unabhängig voneinander Niederalkyl bedeuten, oder
R₄ und R₅ zusammen -(CH₂)₄- oder -(CH₂)₅- bedeuten, und
R₆, R₇, R₈ und R₉ unabhängig voneinander für Wasserstoff, Niederalkyl, Halogen-niederalkyl, Halogen, Hydroxy, Niederalkoxy oder Niederalkylthio stehen, oder
R₇ und R₈ zusammen einen Benzoring bilden, und
B 5-Tetrazolyl oder eine Gruppe -C(=O)-Y bedeutet, wobei
Y Hydroxy, Niederalkoxy, Amino, Niederalkylamino oder Diniederalkylamino bedeutet;
mit der Massgabe, dass A von einer Gruppe -C(R₄R₅)- verschieden ist, wenn B eine Gruppe -C(=O)-Y bedeutet; und Salze davon.

Die Erfindung betrifft vor allen Dingen die Verbindungen der Formel I, worin
R₁ und R₂ unabhängig voneinander Wasserstoff, Niederalkyl, Trifluormethyl, Halogen, Niederalkoxy, Niederalkylthio oder Cyano bedeuten,
X für für eine Gruppe -CH₂-O- (wobei CH₂ mit dem Chinolinrest und O mit der Gruppe Ar verknüpft ist) oder eine Gruppe -CH=CH- steht,
Ar für 1,3-Phenylen steht,
R₃ Wasserstoff oder Niederalkyl bedeutet,
A für eine Gruppe -C(R₄R₅)- oder 1,2-Phenylen steht, wobei R₄ und R₅ unabhängig voneinander C₁-C₃-Alkyl bedeuten,
B 5-Tetrazolyl oder eine Gruppe -C(=O)-Y bedeutet, wobei Y Hydroxy, Niederalkoxy, Amino, Niederalkylamino oder Diniederalkylamino bedeutet;
mit der Massgabe, dass A von einer Gruppe -C(R₄R₅)- verschieden ist, wenn B eine Gruppe -C(=O)-Y bedeutet; und Salze davon.

Die Erfindung betrifft in erster Linie die Verbindungen der Formel I, worin
R₁ und R₂ unabhängig voneinander Wasserstoff oder Halogen bedeuten,
X für für eine Gruppe -CH₂-O- (wobei CH₂ mit dem Chinolinrest und O mit der Gruppe
Ar verknüpft ist) oder eine Gruppe -CH=CH- steht,
Ar für 1,3-Phenylen steht,
R₃ Wasserstoff bedeutet,
A für eine Gruppe -C(C₂H₅)₂- oder 1,2-Phenylen steht, und
B 5-Tetrazolyl oder -COOH bedeutet, wobei
mit der Massgabe, dass A von einer Gruppe -C(C₂H₅)₂- verschieden ist, wenn B eine Gruppe -COOH bedeutet; und pharmazeutisch verwendbare Salze davon.

Als Untergruppen aus einer Gruppe von Verbindungen der Formel I sind jeweils hervorzuheben:
(a) Verbindungen der Formel I, worin A für die Gruppe -C(R₄R₅)- steht und B 5-Tetrazolyl bedeutet; (b) Verbindungen der Formel I, worin A für die Gruppe 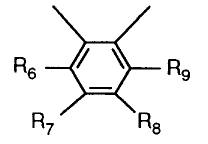 steht und X die Gruppe -CH₂-O- bedeutet;

Die Erfindung betrifft vor allem die in den Beispielen beschriebenen spezifischen Verbindungen und Salze davon.

Die Verbindungen der Formel I können in an sich bekannter Weise hergestellt werden, indem man z.B.
(a) eine Verbindung der Formel II, 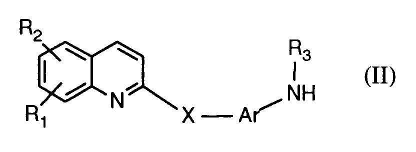 worin R₁,R₂,R₃, X und Ar wie unter Formel I definiert sind, mit einer Verbindung der Formel III, 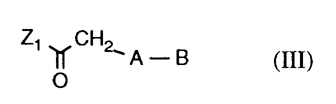 worin die Gruppe -COZ₁ Carboxy oder ein reaktionsfähiges Carboxyderivat bedeutet und A und B wie unter Formel I definiert sind, umsetzt, oder
(b) zur Herstellung von Verbindungen der Formel I, worin X für eine Gruppe -CH₂-O-steht, eine Verbindung der Formel IV, 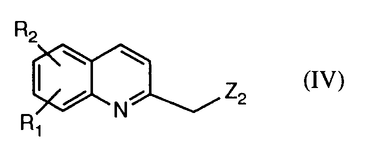 worin Z₂ eine nukleofuge Abgangsgruppe bedeutet und R₁ und R₂ wie unter Formel I definiert sind, mit einer Verbindung der Formel V, 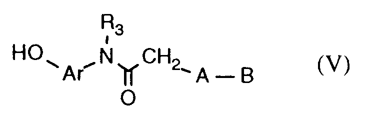 worin R₃, A, B und Ar wie unter Formel I definiert sind, oder einem Salz davon, umsetzt, oder
(c) zur Herstellung von Verbindungen der Formel I, worin B 5-Tetrazolyl bedeutet, eine Verbindung der Formel VII, 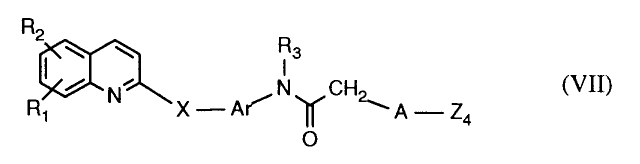 worin Z₄ ein reaktionsfähiges Carboxyderivat, insbesondere Cyano, bedeutet, mit einem ringbildenden Agens, z.B. einem Azid, umsetzt;
   und, wenn erwünscht, eine erhaltene Verbindung der Formel I in eine andere Verbindung der Formel I umwandelt, und/oder, wenn erwünscht, ein erhaltenes Salz in die freie Verbindung oder in ein anderes Salz umwandelt, und/oder, wenn erwünscht, eine erhaltene freie Verbindung der Formel I mit salzbildenden Eigenschaften in ein Salz umwandelt.

Verfahren (a): Die Umsetzung gemäss dem Verfahren (a) entspricht der an sich bekannten Acylierung von primären oder sekundären aromatischen Aminen (bzw. Bildung von Carbonsäureamiden).

Ein reaktionsfähiges Carboxyderivat -COZ₁ ist z.B. ein Carbonsäurehalogenid, etwa ein Säurechlorid, ein Carbonsäureimidazolid, ein Carbonsäureanhydrid oder ein reaktiver Carbonsäureester, z.B. ein p-Nitrophenylester.

Zur Herstellung von Verbindungen der Formel I mit B = COOH kann vorteilhaft als Verbindung der Formel III auch ein inneres Säureanhydrid eingesetzt werden; ein solches inneres Säureanhydrid entspricht einer Verbindung der Formel III, worin die Reste Z₁ und Y [aus -C(=O)-Y] zusammen eine -O-Brücke bilden (z.B. Homophthalsäureanhydrid). Es kann aus der entsprechenden Dicarbonsäure durch Umsetzung z.B. mit Oxalylchlorid oder Acetylchlorid hergestellt werden.

Die Verbindungen der Formel II, worin X für eine Gruppe -CH₂-O- steht, werden z.B. dadurch hergestellt, dass man eine Verbindung der Formel IV [s. Verfahren (b)] mit einer Verbindung der Formel VI 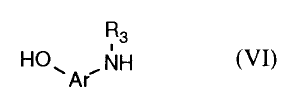
umsetzt.

Die Verbindungen der Formel II, worin X für eine Gruppe -CH=CH- steht, werden z.B. dadurch hergestellt, dass man eine Nitroverbindung der Formel VIII 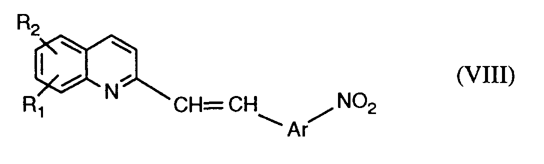
reduziert, z.B. mit Zinn-II-chlorid, und gegebenenfalls noch den Rest R₃ einführt (z.B. via N-Alkylierung). Verbindungen der Formel VIII werden vorzugsweise durch Aldolkondensation aus den entsprechenden 2-Methylchinolinen und Nitroarylaldehyden erhalten.

Die Verbindungen der Formel III sind an sich bekannt oder werden analog zu den bekannten Verbindungen hergestellt.

Verfahren (b): Beim Verfahren (b) wird eine Hydroxy-arylverbindung der Formel V in an sich bekannter Weise mit einem Chinolinmethyl-Derivat der Formel IV alkyliert.

In einer Verbindung der Formel IV bedeutet die nucleofuge Abgangsgruppe Z₂ z.B. Halogen, insbesondere Brom, Iod oder Chlor, oder Sulfonyloxy, z.B. Methyl- oder p-Toluolsulfonyloxy.

Die Verbindungen der Formel IV werden in an sich bekannter Weise beispielsweise aus den entsprechenden Methylchinolinen hergestellt, z.B. durch Halogenierung, etwa mit N-Bromsuccinimid.

Die Verbindungen der Formel V werden z.B. analog zu den Verbindungen der Formel I in Verfahren (a) hergestellt, wobei man anstelle einer Verbindung der Formel II eine Verbindung der Formel VI [s. Verfahren (a)] als Ausgangsmaterial einsetzt.

Verfahren (c): Beim Verfahren (c) werden 5-Tetrazolyl-Verbindungen aus einem Carboxy-Derivat und einem mehrere Stickstoffatome enthaltenden Ringbildner hergestellt.

Z₄ steht vorzugsweise für Cyano, kann aber z.B. auch Carbamoyl (-CONH₂) oder Halogen- bzw. Niederalkoxyiminomethyl [-CH=N-Hal bzw. -CH=N-OAlk (Hal = Halogen, Alk = Niederalkyl)] bedeuten.

Das ringbildende Agens ist vorzugsweise Stickstoffwasserstoffsäure (HN₃) oder ein Salz davon, z.B. Natrium- oder Ammoniumazid. Besonders geeignet ist z.B. auch Tributylzinnazid.

Die Ausgangsverbindungen der Formel VII entsprechen Verbindungen der Formel I mit B = Cyano und werden analog zu letzteren hergestellt, vorzugsweise analog zu Verfahren (a).

Verbindungen der Formel I können auch in andere Verbindungen der Formel I überführt werden.

So kann z.B. eine Verbindung der Formel I, worin R₃ für Wasserstoff steht, am Amidstickstoff alkyliert werden, wobei man eine Verbindung der Formel I erhält, worin R₃ Niederalkyl bedeutet. Die Umsetzung wird in Gegenwart einer geeigneten Base, z.B. NaH oder Natriummethoxid, durchgeführt, und als Alkylierungsmittel dient z.B. eine Verbindung R₃-Z₃, worin Z₃ eine nukleofuge Abgangsgruppe, z.B. Halogen, insbesondere Brom, Iod oder Chlor, oder Sulfonyloxy, z.B. Methyl- oder p-Toluolsulfonyloxy, bedeutet und R₃ wie unter Formel I definiert ist.

Carbonsäuren der Formel I, d.h. Verbindungen der Formel I mit B = COOH, können in an sich bekannter Weise in die entsprechenden Carbonsäure-Derivate der Formel I, worin B für (Niederalkoxy, Amino, Niederalkylamino oder Diniederalkylamino)-carbonyl steht, umgewandelt werden. Umgekehrt können die genannten Carbonsäure-Derivate in an sich bekannter Weise, z.B. durch Umsetzung mit LiOH/Tetrahydrofuran/Wasser/Methanol, etwa bei einer Temperatur von 50°, in die freien Carbonsäuren der Formel I überführt werden. Weiterhin können z.B. auch Verbindungen der Formel I, worin B Niederalkoxycarbonyl bedeutet, - etwa durch Umsetzung mit Ammoniak, Alkylaminen oder Dialkylaminen - in die entsprechenden Carbonsäure-Derivate der Formel I, worin B für (Amino, Niederalkylamino oder Dialkylamino)-carbonyl steht, umgewandelt werden.

Wenn irgendwelche Zwischenprodukte störende reaktionsfähige Gruppen, z.B. Carboxy-, Hydroxy-, Mercapto- oder Aminogruppen, enthalten, können diese vorübergehend durch leichtabspaltbare Schutzgruppen geschützt werden. Die Auswahl von geeigneten Schutzgruppen, ihre Einführung und Entfernung sind an sich bekannt und sind z.B. in J.F.W. McOmie, Protective Groups in Organic Chemistry, Plenum Press, London, New York 1973 beschrieben.

Salze von Verbindungen I können in an sich bekannter Weise hergestellt werden. So erhält man beispielsweise Säureadditionssalze von Verbindungen I durch Behandeln mit einer geeigneten Säure oder einem geeigneten Ionenaustauscherreagens und Salze mit Basen durch Behandeln mit einer geeigneten Base oder einem geeigneten Ionenaustauscherreagens. Salze von Verbindungen der Formel I können in üblicher Weise in die freien Verbindungen I überführt werden, Säureadditionssalze z.B. durch Behandeln mit einem geeigneten basischen Mittel oder einem geeigneten Ionenaustauscherreagens und Salze mit Basen z.B. durch Behandeln mit einer geeigneten Säure oder einem geeigneten Ionenaustauscherreagens.

Salze von Verbindungen I können in an sich bekannter Weise in andere Salze von Verbindungen I umgewandelt werden, Säureadditionssalze beispielsweise in andere Säureadditionssalze, z.B. durch Behandeln eines Salzes einer anorganischen Säure, wie eines Hydrochlorids, mit einem geeigneten Metallsalz, wie einem Natrium-, Barium- oder Silbersalz, einer Säure, z.B. mit Silberacetat, in einem geeigneten Lösungsmittel, in welchem ein sich bildendes anorganisches Salz, z.B. Silberchlorid, unlöslich ist und damit aus dem Reaktionsgemisch ausscheidet.

Je nach Verfahrensweise bzw. Reaktionsbedingungen können die Verbindungen I mit salzbildenden Eigenschaften in freier Form oder in Form von Salzen erhalten werden.

Infolge der engen Beziehung zwischen der Verbindung I in freier Form und in Form ihrer Salze sind im vorausgegangenen und nachfolgend unter der freien Verbindung I bzw. ihren Salzen sinn- und zweckgemäss gegebenenfalls auch die entsprechenden Salze bzw. die freie Verbindung I zu verstehen.

Die Verbindungen I einschliesslich ihrer Salze von salzbildenden Verbindungen können auch in Form ihrer Hydrate erhalten werden und/oder andere, beispielsweise gegebenenfalls zur Kristallisation von in fester Form vorliegenden Verbindungen verwendete, Lösungsmittel einschliessen.

Die Verbindungen I und ihre Salze können, je nach Wahl der Ausgangsstoffe und Arbeitsweisen, in Form eines der möglichen Isomeren oder als Gemisch derselben vorliegen. Dabei sind als reine Isomere z.B. reine Diastereomere erhältlich. Entsprechend können als Isomerengemische z.B. Diastereomerengemische vorliegen. Verfahrensgemäss oder anderweitig erhältliche Isomerengemische von Verbindungen I in freier Form oder in Salzform können in üblicher Weise in die Komponenten aufgetrennt werden, z.B. aufgrund der physikalisch-chemischen Unterschiede der Bestandteile in bekannter Weise durch fraktionierte Kristallisation, Destillation und/oder Chromatographie. Vorteilhaft isoliert man das wirksamere Isomere.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Schritte durchführt oder einen Ausgangsstoff in Form eines Derivates bzw. Salzes verwendet oder insbesondere unter den Reaktionsbedingungen bildet.

Beim Verfahren der vorliegenden Erfindung werden vorzugsweise solche Ausgangsstoffe und Zwischenprodukte, jeweils in freier Form oder in Salzform, verwendet, welche zu den eingangs als besonders wertvoll geschilderten Verbindungen I bzw. deren Salzen führen. Neue Ausgangsstoffe und Zwischenprodukte, jeweils in freier Form oder in Salzform, für die Herstellung der Verbindungen I bzw. ihrer Salze, ihre Verwendung und Verfahren zu ihrer Herstellung bilden ebenfalls einen Gegenstand der Erfindung.

Die Erfindung betrifft ebenfalls die Verwendung der Verbindungen I und ihrer pharmazeutisch verwendbaren Salze zur Behandlung von allergischen Zuständen und Erkrankungen,vorzugsweise in Form von pharmazeutisch verwendbaren Zubereitungen, insbesondere in einem Verfahren zur therapeutischen Behandlung des tierischen oder menschlichen Körpers, sowie ein solches Behandlungsverfahren.

Die Erfindung betrifft gleichfalls pharmazeutische Präparate, die eine Verbindung I oder ein pharmazeutisch verwendbares Salz davon als Wirkstoff enthalten, sowie Verfahren zu ihrer Herstellung. Bei diesen pharmazeutischen Präparaten handelt es sich um solche zur enteralen, wie oralen, ferner rektalen, Verabreichung, um solche zur parenteralen Verabreichung, um solche zur lokalen Verabreichung und vor allem um solche zur Inhalationsverabreichung an Warmblüter, vor allem an Menschen, wobei der pharmakologische Wirkstoff allein oder zusammen mit üblichen pharmazeutischen Hilfsstoffen enthalten ist. Die pharmazeutischen Präparate enthalten (in Gewichtsprozenten) z.B. von etwa 0,001 % bis 100 %, vorzugsweise von etwa 0,1 % bis etwa 50 %, des Wirkstoffs.

Pharmazeutische Präparate zur enteralen bzw. parenteralen Verabreichung sind z.B. solche in Dosiseinheitsformen, wie Dragées, Tabletten, Kapseln oder Suppositorien, ferner Ampullen. Diese werden in an sich bekannter Weise, z.B. mittels konventioneller Misch-, Granulier-, Dragier-, Lösungs- oder Lyophilisierungsverfahren, hergestellt. So kann man pharmazeutische Präparate zur oralen Anwendung erhalten, indem man den Wirkstoff mit festen Trägerstoffen kombiniert, ein erhaltenes Gemisch gegebenenfalls granuliert und das Gemisch bzw. Granulat, wenn erwünscht oder notwendig nach Zugabe von geeigneten Hilfsstoffen, zu Tabletten oder Dragée-Kernen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister, unter Verwendung z.B. von Mais-, Weizen-, Reis- oder Kartoffelstärke, Gelatine, Tragaganth, Methylcellulose und/oder Polyvinylpyrrolidon, und, wenn erwünscht, Sprengmittel, wie die obengenannten Stärken, ferner Carboxymethylstärke, quervernetztes Polyvinylpyrrolidon, Agar oder Alginsäure oder ein Salz davon, wie Natriumalginat. Hilfsmittel sind in erster Linie Fliessregulier- und Schmiermittel, z.B. Kieselsäure, Talk, Stearinsäure oder Salze davon, wie Magnesium- oder Calciumstearat, und/oder Polyäthylenglykol. Dragée-Kerne werden mit geeigneten, gegebenenfalls Magensaft-resistenten, Ueberzügen versehen, wobei man u.a. konzentrierte Zuckerlösungen, welche gegebenfalls arabischen Gummi, Talk, Polyvinylpyrrolidon, Polyäthylenglykol und/oder Titandioxid enthalten, Lacklösungen in geeigneten organischen Lösungsmitteln oder Lösungsmittelgemischen oder, zur Herstellung von Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Weitere oral anwendbare pharmazeutische Präparate sind Steckkapseln aus Gelatine sowie weiche, geschlossene Kapseln aus Gelatine und einem Weichmacher wie Glycerin oder Sorbitol. Die Steckkapseln können den Wirkstoff in Form eines Granulats, z.B. im Gemisch mit Füllstoffen, wie Lactose, Bindemitteln, wie Starken, und/oder Gleitmitteln, wie Talk oder Magnesiumstearat, und gegebenenfalls Stabilisatoren, enthalten. In weichen Kapseln ist der Wirkstoff vorzugsweise in geeigneten Flüssigkeiten, wie fetten Oelen, Paraffinöl oder flüssigen Polyäthylenglykolen, gelöst oder suspendiert, wobei ebenfalls Stabilisatoren zugefügt sein können.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffinkohlenwasserstoffe, Polyäthylenglykole oder höhere Alkanole. Ferner können auch Gelatine-Rektalkapseln verwendet werden, die eine Kombination des Wirkstoffs mit einem Grundmassenstoff enthalten. Als Grundmassenstoffe kommen z.B. flüssige Triglyceride, Polyäthylenglykole oder Paraffinkohlenwasserstoffe in Frage.

Zur parenteralen Verabreichung eignen sich in erster Linie wässrige Lösungen eines Wirkstoffs in wasserlöslicher Form, z.B. eines wasserlöslichen Salzes, ferner Suspensionen des Wirkstoffs, wie entsprechende ölige Injektionssuspensionen, wobei man geeignete lipophile Lösungsmittel oder Vehikel, wie fette Oele, z.B. Sesamöl, oder synthetische Fettsäureester, z.B. Aethyloleat oder Triglyceride, verwendet, oder wässrige Injektionssuspensionen, welche viskositätserhöhende Stoffe, z.B. Natriumcarboxymethylcellulose, Sorbit und/oder Dextran, und gegebenenfalls auch Stabilisatoren enthalten.

Pharmazeutische Präparate zur lokalen Verabreichung sind z.B. für die topische Behandlung der Haut Lotionen, Crèmes und Salben, d. h. flüssige oder semifeste Oel-in-Wasser- oder Wasser-in-Oel-Emulsionen, Fettsalben, die wasserfrei sind, Pasten, d. h. Crèmes und Salben mit sekretabsorbierenden Puderbestandteilen, Gele, die wässrig, wasserarm oder wasserfrei sind und aus quellbaren, gelbildenden Materialien bestehen, Schäume, d. h. in Aerosolform vorliegende flüssige Oel-in-Wasser-Emulsionen, welche aus Druckbehältern verabreicht werden, und eine wässrig-ethanolische Grundlage aufweisende Tinkturen, welche jeweils weitere übliche pharmazeutische Hilfsstoffe, wie Konservierungsmittel, enthalten können. Für die lokale Behandlung der Augen eignen sich z.B. Augentropfen, welche den Wirkstoff in steriler wässriger oder öliger Lösung enthalten, und Augensalben, die vorzugsweise ebenfalls in steriler Form hergestellt werden. Für die lokale Behandlung der Nase sind z.B. Sprays, ähnlich den weiter unten beschriebenen für die Behandlung der Atemwege, grobe Puder, die durch schnelles Inhalieren durch die Nasenlöcher verabreicht werden, und vor allem Nasentropfen, welche den Wirkstoff in wässriger oder öliger Lösung enthalten, geeignet. Für die lokale Behandlung der Mundhöhle eignen sich z.B. Lutschbonbons und Pastillen, welche den Wirkstoff in einer z.B. aus Zucker und Gummiarabikum oder Tragantgummi gebildeten inerten Masse enthalten, welcher Geschmacksstoffe beigegeben sein können. Die Herstellung der pharmazeutischen Präparate zur lokalen Verabreichung erfolgt in an sich bekannter Weise durch Vermischung des Wirkstoffs mit den pharmazeutischen Hilfsstoffen, z.B. durch Lösen oder Suspendieren des Wirkstoffs in der Grundlage oder in einem Teil davon, falls notwendig. Zur Herstellung von Emulsionen, in denen der Wirkstoff in einer der flüssigen Phasen gelöst ist, wird der Wirkstoff in der Regel vor der Emulgierung in dieser gelöst; zur Herstellung von Suspensionen, in denen der Wirkstoff in der Emulsion suspendiert ist, wird der Wirkstoff nach der Emulgierung mit einem Teil der Grundlage vermischt und dann dem Rest der Formulierung beigegeben.

Pharmazeutische Präparate zur Inhalationsverabreichung sind solche, in denen der Wirkstoff in mikronisierter Form vorliegt, d. h. in denen die Partikelgrösse des Wirkstoffs weniger als 20 µm, insbesondere weniger als 10 µm und vorteilhaft weniger als 5 µm, beträgt, z.B. mikronisierte Pulver und Aerosole, die in Form von Sprays verabreicht werden. Die mikronisierten Pulver enthalten den Wirkstoff allein oder zusammen mit einem inerten Trägerstoff, wie Lactose, vorteilhaft zusammen mit einem der nachstehend erwähnten Treibmittel. Aerosole sind Lösungen, Suspensionen oder Emulsionen des Wirkstoffs in einer geeigneten, pharmazeutisch verwendbaren, flüssigen Phase, wie in Ethanol oder Wasser oder einem entsprechenden Gemisch, können nach Bedarf auch andere pharmazeutische Hilfsstoffe, wie nicht-ionische oder anionische oberflächenaktive Mittel, Emulgatoren und Stabilisatoren, und/oder Wirkstoffe anderer Art aufweisen und enthalten ein Treibmittel, z.B. ein inertes Gas, wie Butan, unter erhöhtem Druck oder insbesondere eine leicht flüchtige, vorzugsweise unter normalem Druck unterhalb der üblichen Raumtemperatur (z.B. zwischen etwa -30°C und etwa +10°C) siedende, Flüssigkeit, wie ein mindestens teilweise fluoriertes polyhalogeniertes Niederalkan, oder ein Gemisch solcher Flüssigkeiten. Zur Herstellung der pharmazeutischen Präparate in zur Inhalationsverabreichung fertiger Form wird eine entsprechende pharmazeutische Zusammensetzung zusammen mit dem Treibmittel in geeignete Behälter, wie Flacons oder Druckflaschen, abgefüllt, welche mit einer geeigneten Versprühungseinrichtung, z.B. einem Ventil, versehen sind. Das Ventil ist vorzugsweise als Dosierungsventil konstruiert, welches bei Betätigung eine vorbestimmte Menge des Behälterinhalts, entsprechend einer vorbestimmten Dosis des Wirkstoffs, freigibt. Bei der Herstellung der fertigen Arzneimittelform kann man auch so vorgehen, dass entsprechende Mengen der pharmazeutischen Zusammensetzung und des Treibmittels separat in die Behälter abgefüllt werden und erst dort zur Vermischung kommen.

Die Dosierung des Wirkstoffs kann von verschiedenen Faktoren, wie Wirksamkeit und Wirkungsdauer des Wirkstoffs, Stärke der zu behandelnden Krankheit bzw. ihrer Symptome, Applikationsweise, Warmblüter-Spezies, -Geschlecht, -Alter, -Gewicht und/oder individuellem Zustand des Warmblüters, abhängen. Im Normalfall ist für einen etwa 75 kg schweren Warmblüter eine, z.B. orale, Tagesdosis von etwa 1 mg bis etwa 150 mg, insbesondere von etwa 2,5 bis etwa 50 mg zu veranschlagen. Diese kann z.B. als Einmaldosis oder in mehreren Teildosen, z.B. solchen von 10 bis 50 mg, verabreicht werden.

Die nachfolgenden Beispiele illustrieren die vorstehend beschriebene Erfindung. Temperaturen sind in Grad Celsius angegeben [DMF = Dimethylformamid, DMSO = Dimethylsulfoxid, Ethylacetat = Essigsäureethylester; Hexan meint ein Isomerengemisch verschiedener Hexane (Fa. Fluka)].

### Beispiel 1: 5-{3-[3-(7-Fluor-2-chinolinylmethoxy)phenylamino]-1,1-diethyl-3-oxopropy}-1H-tetrazol

Eine Mischung von 0.25g 4-[3-(7-Fluor-2-chinolinylmethoxy)phenylamino]-2,2-diethyl-4-oxobutansäurenitril und 0.3g Tributylzinnazid wird in 10 ml m-Xylol für 6h am Rückfluss gekocht, auf Raumtemperatur abgekühlt, mit 15ml 2N KOH versetzt und 0.5h heftig gerührt. Nach dem Abtrennen der organischen Phase wird die wässrige Phase noch zweimal mit Diethylether/Methylenchlorid 6:1 extrahiert. Durch Zugabe von 1N HCl wird der pH-Wert der wässrigen Phase auf ca. 1 eingestellt. Durch dreimalige Extraktion mit Methylenchlorid wird die Titelverbindung extrahiert. Die vereinigten Methylenchloridextrakte werden mit ges. Kochsalzlösung gewaschen, getrocknet (Magnesiumsulfat) und am Rotationsverdampfer eingedampft. Der Rückstand wird an Kieselgel mit Ethylacetat chromatographiert. Die Titelverbindung wird auf diese Weise in Form eines weissen amorphen Feststoffes erhalten; ¹H-NMR, 400MHz, DMSO, delta (ppm): 7.43(d, 1H), 8.08(dd, 1H), 7.75 (dd, 1H), 7.62(delta, 1H), 7.55(m, 1H), 7.38(m, 1H), 7.15(dd, 1H), 7.00(m, 1H), 6.67(dd, 1H), 5.30(s, 2H), 2.80(s, 2H), 1.80(q, 4H), 0.68(t, 6H).

Die Ausgangsmaterialien können wie folgt hergestellt werden:
a) 4-[3-(7-Fluor-2-chinolinylmethoxy)phenylamino]-2,2-diethyl-4-oxobutansäurenitril: Eine Mischung von 0.536g 3-(7-Fluor-2-chinolinylmethoxy)anilin, 0.31g 3-Cyano-3,3-diethyl-propancarbonsäure, 0.4g N-Ethyl-N-(3-dimethylaminopropyl)carbodiimid-hydrochlorid und 0.256g 4-Dimethylamino-pyridin in 15ml Methylenchlorid wird unter Argon 4Std. bei Raumtemperatur gerührt. Anschliessend wird das Reaktionsgemisch mit 100ml Ethylacetat versetzt, und die entstandene trübe Lösung wird dreimal mit Wasser und einmal mit gesättigter Kochsalzlösung gewaschen, getrocknet (Magnesiumsulfat) und eingedampft. Auf diese Weise wird die Titelverbindung in Form eines weissen Feststoffes erhalten. ¹H-NMR, 200MHz, CDCl₃, delta (ppm): 8.18(d, 1H), 7.81(dd, 1H), 7.67(m, 2H), 7.17-7.45(m, 4H), 7.03(dd, 1H), 6.78(dd, 1H), 5.33(s, 2H), 2.60(s, 2H), 1.80(q, 4H), 1.10(t, 6H).
b) 3-Cyano-3,3-diethyl-propancarbonsäure: 2.2g 3-Cyano-3,3-diethyl-propancarbonsäure-ethylester werden in 25ml 4N methanolischer NaOH gelöst und für 20Min. gerührt Durch Zugabe von 1N Salzsäure wird ein pH-Wert von ca. 2 eingestellt. Diese Lösung wird zweimal mit Ethylacetat extrahiert, die vereinigten organischen Phasen werden mit ges. Kochsalzlösung gewaschen, getrocknet (Magnesiumsulfat) und eingedampft. Die Titelverbindung wird als farbloses Oel erhalten. ¹H-NMR, 200MHz, CDCl₃, delta (ppm): 2.65(s, 2H), 1.75(q, 4H), 1.05(t, 6H).
c) 3-Cyano-3,3-diethyl-propancarbonsäure-ethylester: Eine Mischung aus 5g 3-Pentyliden-malonsäure-diethylester [Liebigs Ann. Chem. 1981, 748], 3.25g Kaliumcyanid, 16g Ethanol und 12g Wasser werden 6Std. am Rückfluss gekocht, und anschliessend mit 50ml Wasser verdünnt. Die Titelverbindung wird durch zweimalige Extraktion mit Diethylether in Form eines farblosen Oels gewonnen. ¹H-NMR, 200MHz, CDCl₃, delta (ppm): 4.20(q,2H), 2.65(s, 2H), 1.75(q, 4H), 1.30(t, 3H), 1.05(t, 6H).
d) 2-Brommethyl-7-fluorchinolin: Eine Lösung von 130g 2-Methyl-7-fluorchinolin [Z. Song et al., J. Heterocyclic Chem. 30 (1993) 17-21] in 1500ml CCl₄ wird mit 215g N-Bromsuccinimid und 210mg Azoisobutyronitril versetzt. Die resultierende Suspension wird 27 Std. am Rückfluss gekocht, filtriert und eingedampft. Der Rückstand wird an Kieselgel mit Hexat/Ethylacetat 9:1 bis 7:3 chromatographiert. Die Titelverbindung wird in Form von farblosen Kristallen vom Smp. 101-102^{o} erhalten.
e) 3-(7-Fluor-2-chinolinylmethoxy)anilin: Eine Lösung von 13.6g 3-Aminophenol in 300ml Aceton wird mit19.9g Natriumcarbonat versetzt und 15 Min. bei 20^{o} gerührt. Dieses Gemisch wird mit 30g 2-Brommethyl-7-fluorchinolin, 40.7g Cäsiumcarbonat und 1g Kaliumiodid versetzt und 3 Std. am Rückfluss gekocht. Das Reaktionsgemisch wird filtriert, der Niederschlag mit Aceton gewaschen und das Filtrat eingedampft. Der Rückstand wird in Methylenchlorid aufgenommen, mit Wasser gewaschen, über Natriumsulfat getrocknet und eingedampft. Das resultierende orangefarbene Oel wird an 2000g Kieselgel mit Hexan/Ethylacetat 3:1 chromatographiert. Die Titelverbindung wird so als gelber Feststoff vom Smp. 88-89^{o} erhalten.

### Beispiel 2: 5-{3-[3-(7-Fluor-2-chinolinylvinyl)phenylamino]-1,1-diethyl-3-oxopropyl}-1H-tetrazol (= 5-{3-[3-[2-(7-Fluor-2-chinolinyl)ethen]phenylamino]-1,1-diethyl-3-oxopropyl}-1H-tetrazol)

Die Titelverbindung wird ausgehend von 4-[3-(7-Fluor-2-chinolinylvinyl)phenylamino]-2,2-diethyl-4-oxobutansäurenitril in der gleichen Weise wie unter Beispiel 1 beschrieben hergestellt. Sie wird in Form eines gelben amorphen Feststoffes erhalten. ¹H-NMR, 400MHz, DMSO, delta (ppm): 8.45(d, 1H), 8.06(dd, 1H), 7.95 (m, 2H), 7.86(d, 1H), 7.77(dd, 1H), 7.38(m, 1H), 7.5- 7.38(m, 5H), 2.93(s, 2H), 1.95(m, 4H), 0.78(t, 6H).

Die Ausgangsmaterialien können wie folgt hergestellt werden:
a) 4-[3-(7-Fluor-2-chinolinylvinyl)phenylamino]-2,2-diethyl-4-oxobutansäurenitril: Die Verbindung wird in analoger Weise wie unter Beispiel 1a) beschrieben aus 3-(7-Fluor-2-chinolinylvinyl)anilin hergestellt. ¹H-NMR, 200MHz, DMSO, delta (ppm): 8.45(d, 1H), 8.1-7.8(m, 4H), 7.975 (dd, 1H), 7.55- 7.35(m, 5H), 2.70(s, 2H), 1.78(m, 4H), 1.0(t, 6H).
b) 3-(7-Fluor-2-chinolinylvinyl)anilin: Zu einer Suspension von 25g 3-(7-Fluor-2-chinolinylvinyl)nitrobenzol in 330ml Ethanol wird eine Lösung von 58.7g Zinn-II-chlorid in 330ml Ethanol zugetropft. Diese Mischung wird 7 Std. am Rückfluss gekocht, dann auf Raumtemperatur abgekühlt und durch Zugabe von 2N Natronlauge auf pH = 13 eingestellt. Die Titelverbindung wird zweimal mit je 200ml Methylenchlorid extrahiert. Die vereinigten organischen Phasen werden mit Wasser gewaschen, mit Magnesiumsulfat getrocknet und am Rotationsverdampfer eingedampft. Der Rückstand wird an Kieselgel mit Hexan/Ethylacetat 1:1 chromatographiert. Auf diese Weise wird die Titelverbindung in Form gelblicher Kristalle vom Smp. 143-147^{o} erhalten.
c) 3-(7-Fluor-2-chinolinylvinyl)nitrobenzol: Ein Gemisch aus 19.5g 2-Methyl-7-fluorchinolin [Z. Song et al., J. Heterocyclic Chem. 30 (1993) 17-21], 18.2g 3-Nitrobenzaldehyd und 200ml Acetanhydrid wird während 16 Std. bei 100^{o} gerührt. Anschliessend wird das Reaktionsgemisch auf 0^{o} abgekühlt. Die ausgefallenen hellgelben Kristalle werden abfiltriert, mit Diethylether gewaschen und an der Luft getrocknet. Die auf diese Weise hergestellte Titelverbindung hat einen Smp. von 158-159^{o}.

### Beispiel 3: 2-[3-(7-Fluor-2-chinolinylmethoxy)phenylaminocarbonylmethyl]-benzoesäure

1.34g 3-(7-Fluor-2-chinolinylmethoxy)anilin und 0.85g Homophtalsäureanhydrid werden in 30ml Toluol suspendiert und während 45 min. am Rückfluss gekocht. Die abgekühlte Suspension wird abgesaugt und aus Ethanol kristallisiert. Man erhält farblose Kristalle vom Smp. 219-220^{o}.

### Beispiel 4: 5-{2-[3-(7-Fluor-2-chinolinylmethoxy)phenylaminocarbonylmethyl]-phenyl}-1H-tetrazol

3-(7-Fluor-2-chinolinylmethoxy)anilin und 2-Cyanophenylessigsäure (Bull.Soc.Chim. France 1968, 8, 3403) werden in analoger Weise wie in Beispiel 1a und 1 beschrieben (2 Stufen) zur Titelverbindung umgesetzt.

### Beispiele A bis G: Pharmazeutische Präparate

Unter dem Ausdruck "Wirkstoff" ist nachstehend eine Verbindung der Formel I, in freier Form oder in Form eines pharmazeutisch verwendbaren Salzes, zu verstehen, insbesondere eine solche Verbindung, die als Produkt in einem der vorstehenden Beispiele beschrieben ist.

### Beispiel A: Eine treibmittelhaltige, feststoffaerosolbildende Inhalationssuspension, enthaltend 0,1 Gewichtsprozent Wirkstoff:

| Zusammensetzung | Gewichtsprozent |
|---|---|
| Wirkstoff, mikronisiert | 0,1 |
| Sorbitantrioleat | 0,5 |
| Treibmittel A (Trichlortrifluorethan) | 4,4 |
| Treibmittel B (Dichlordifluormethan und | 15,0 |
| 1,2-Dichlortetrafluorethan) | 80,0 |

Der Wirkstoff wird unter Feuchtigkeitsausschluss mit Hilfe eines üblichen Homogenisators unter Zusatz des Sorbitantrioleats im Trichlortrifluorethan suspendiert und die Suspension in einen mit einem Dosierventil versehenen Aerosolbehälter abgefüllt.

Der Behälter wird verschlossen und unter Druck mit dem Treibmittel B aufgefüllt.

### Beispiel B: Eine zur Inhalation geeignete, etwa zweiprozentige, wässrige Lösung des Wirkstoffs in Form seines Natrium- oder Kalium-salzes:

| Zusammensetzung | |
|---|---|
| Wirkstoff (K- oder Na-Salz) | 2000 mg |
| Ethylendiamintetraessigsäure-Dinatriumsalz | 10 mg |
| Benzalkoniumchlorid | 10 mg |
| Wasser, frisch destilliert | ad 100 ml |
| Treibmittel | nach Bedarf |

Der Wirkstoff wird in etwa 60 ml frisch destilliertem Wasser gelöst und der Stabilisator (Ethylendiamintetraessigsäure-Dinatriumsalz) und das Konservierungsmittel (Benzalkoniumchlorid) werden hinzugegeben. Nach vollständiger Auflösung aller Komponenten wird die erhaltene Lösung auf 100 ml aufgefüllt und in Druckfläschchen abgefüllt. Die Fläschchen werden gasdicht verschlossen. Das Treibmittel wird nach Bedarf gasförmig unter Druck oder in flüssiger Form zugegeben.

### Beispiel C: Eine Salbe, enthaltend 0,05 Gewichtsprozent Wirkstoff:

| Zusammensetzung | Gewichtsprozent |
|---|---|
| Wirkstoff | 0,05 |
| Vaseline | 45,00 |
| Paraffinöl | 19,60 |
| Cetylalkohol | 5,00 |
| Bienenwachs | 5,00 |
| Sorbitan-sesquioleat | 5,00 |
| p-Hydroxybenzoesäureester | 0,20 |
| Wasser, entmineralisiert | 20,15 |

Die Fettstoffe und Emulgatoren werden zusammengeschmolzen. Das Konservierungsmittel wird in Wasser gelöst und die Lösung in die Fettschmelze bei erhöhter Temperatur einemulgiert. Nach dem Erkalten wird eine Suspension des Wirkstoffs in einem Teil der Fettschmelze in die Emulsion eingearbeitet.

### Beispiel D: Tabletten, enthaltend je 50 mg Wirkstoff:

| Zusammensetzung (10000 Tabletten) | |
|---|---|
| Wirkstoff | 500,0 g |
| Lactose | 500,0 g |
| Kartoffelstärke | 352,0 g |
| Gelatine | 8,0 g |
| Talk | 60,0 g |
| Magnesiumstearat | 10,0 g |
| Siliciumdioxid (hochdispers) | 20,0 g |
| Ethanol | q.s. |

Der Wirkstoff wird mit der Lactose und 292 g der Kartoffelstärke vermischt und die Mischung mit einer ethanolischen Lösung der Gelatine befeuchtet und durch ein Sieb granuliert. Nach dem Trocknen mischt man den Rest der Kartoffelstärke, das Magnesiumstearat, den Talk und das Siliciumdioxid zu und presst das Gemisch zu Tabletten von je 145 mg Gewicht und 50 mg Wirkstoffgehalt, die gewünschtenfalls mit Teilkerben zur feineren Anpassung der Dosierung versehen sein können.

### Beispiel E: Lacktabletten, enthaltend je 100 mg Wirkstoff:

| Zusammensetzung (1000 Lacktabletten) | |
|---|---|
| Wirkstoff | 100,0 g |
| Lactose | 100,0 g |
| Maisstärke | 70,0 g |
| Talk | 8,5 g |
| Calciumstearat | 1,5 g |
| Hydroxypropylmethylcellulose | 2,36 g |
| Schellack | 0,64 g |
| Wasser | q.s. |
| Dichlormethan | q.s. |

Der Wirkstoff, die Lactose und 40 g der Maisstärke werden gemischt. Das Gemisch wird mit einem Kleister, hergestellt aus 15 g Maisstärke und Wasser (unter Erwärmen), befeuchtet und granuliert. Das Granulat wird getrocknet und der Rest der Maisstärke, der Talk und das Calciumstearat werden mit dem Granulat vermischt. Das Gemisch wird zu Tabletten (Gewicht: je 280 mg) verpresst und diese werden mit einer Lösung der Hydroxypropylmethylcellulose und des Schellacks in Dichlormethan lackiert (Endgewicht der Lacktabletten: je 283 mg).

### Beispiel F: Gelatinesteckkapseln, enthaltend je 100 mg Wirkstoff:

| Zusammensetzung (1000 Kapseln) | |
|---|---|
| Wirkstoff | 100,0 g |
| Lactose | 250,0 g |
| mikrokristalline Cellulose | 30,0 g |
| Natriumlaurylsulfat | 2,0 g |
| Magnesiumstearat | 8,0 g |

Das Natriumlaurylsulfat wird durch ein Sieb mit einer Maschenweite von 0,2 mm zu dem lyophilisierten Wirkstoff hinzugesiebt. Beide Komponenten werden innig gemischt. Dann wird zunächst die Lactose durch ein Sieb mit einer Maschenweite von 0,6 mm und anschliessend die mikrokristalline Cellulose durch ein Sieb mit einer Maschenweite von 0,9 mm hinzugesiebt. Dann werden alle vier Komponenten 10 Minuten innig gemischt. Zuletzt wird das Magnesiumstearat durch ein Sieb mit einer Maschenweite von 0,8 mm hinzugesiebt. Nach weiterem Mischen (3 Minuten) werden je 390 mg der erhaltenen Formulierung in Gelatinesteckkapseln der Grösse 0 abgefüllt.

### Beispiel G: Eine Injektions- oder Infusionslösung, enthaltend 5 mg Wirkstoff je 2,5 ml-Ampulle:

| Zusammensetzung (1000 Ampullen) | |
|---|---|
| Wirkstoff | 5,0 g |
| Natriumchlorid | 22,5 g |
| Phosphatpufferlösung (pH: 7,4) | 300,0 g |
| entmineralisiertes Wasser | ad 2500,0 ml |

Der Wirkstoff und das Natriumchlorid werden in 1000 ml entmineralisiertem Wasser gelöst Die Lösung wird durch ein Mikrofilter filtriert. Man versetzt das Filtrat mit der Phosphatpufferlösung und füllt das Gemisch mit entmineralisiertem Wasser auf 2500 ml auf. Zur Herstellung von Dosiseinheitsformen werden je 2,5 ml des Gemisches in Glasampullen abgefüllt, die dann je 5 mg Wirkstoff enthalten.

## Patentansprüche

1. Verbindungen der Formel I, 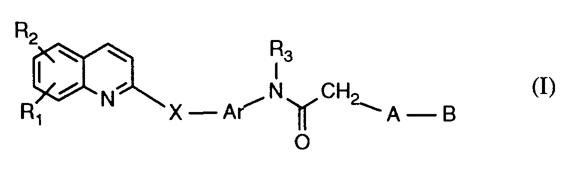 worin
R₁ und R₂ unabhängig voneinander Wasserstoff, Niederalkyl, Halogen-niederalkyl, Arylniederalkyl, Cycloalkyl, Halogen, Hydroxy, Niederalkoxy, Halogen-niederalkoxy, Arylniederalkoxy, Acyloxy, Mercapto, Niederalkyl(-thio, -sulfinyl oder -sulfonyl), Amino, Niederalkylamino, Diniederalkylamino, Acylamino, Nitro, Acyl, Carboxy, Niederalkoxycarbonyl, Aminocarbonyl, N-Niederalkylaminocarbonyl, N,N-Diniederalkylaminocarbonyl oder Cyano bedeuten, oder
R₁ und R₂ zusammen -(CH₂)ₘ-, worin m für 3, 4 oder 5 steht, bedeuten,
X für eine Gruppe -CH₂-O- (wobei CH₂ mit dem Chinolinrest und O mit der Gruppe Ar verknüpft ist) oder eine Gruppe -CH=CH- steht,
Ar für 1,3-Phenylen oder 2,7-Naphthylen steht,
R₃ Wasserstoff oder Niederalkyl bedeutet,
A für eine Gruppe -C(R₄R₅)- oder eine Gruppe 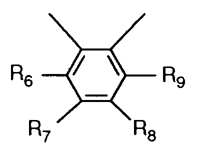 steht, wobei
R₄ und R₅ unabhängig voneinander Niederalkyl bedeuten, oder
R₄ und R₅ zusammen -(CH₂)ₙ-, worin n für 3, 4, 5 oder 6 steht, bedeuten, und
R₆, R₇, R₈ und R₉ unabhängig voneinander für Wasserstoff, Niederalkyl, Halogen-niederalkyl, Halogen, Hydroxy, Niederalkoxy oder Niederalkylthio stehen, oder
R₇ und R₈ zusammen einen Benzoring bilden, und
B 5-Tetrazolyl oder eine Gruppe -C(=O)-Y bedeutet, wobei
Y Hydroxy, Niederalkoxy, Amino, Niederalkylamino oder Diniederalkylamino bedeutet;
mit der Massgabe, dass A von einer Gruppe -C(R₄R₅)- verschieden ist, wenn B eine Gruppe -C(=O)-Y bedeutet,
und Salze davon.

2. Verbindungen der Formel I gemäss Anspruch 1, worin
R₁ und R₂ unabhängig voneinander Wasserstoff, Niederalkyl, Halogen-niederalkyl, Phenylniederalkyl, Halogen, Hydroxy, Niederalkoxy, Halogen-niederalkoxy, Phenylniederalkoxy, Niederalkyl-(thio, sulfinyl oder sulfonyl), Nitro, Niederalkanoyl oder Cyano bedeuten, oder
R₁ und R₂ zusammen -(CH₂)₃- oder -(CH₂)₄- bedeuten,
X für für eine Gruppe -CH₂-O- (wobei CH₂ mit dem Chinolinrest und O mit der Gruppe Ar verknüpft ist) oder eine Gruppe -CH=CH- steht,
Ar für 1,3-Phenylen oder 2,7-Naphthylen steht,
R₃ Wasserstoff oder Niederalkyl bedeutet,
A für eine Gruppe -C(R₄R₅)- oder eine Gruppe 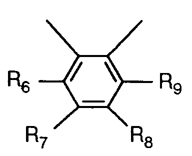 steht, wobei
R₄ und R₅ unabhängig voneinander Niederalkyl bedeuten, oder
R₄ und R₅ zusammen -(CH₂)₄- oder -(CH₂)₅- bedeuten, und
R₆, R₇, R₈ und R₉ unabhängig voneinander für Wasserstoff, Niederalkyl, Halogen-niederalkyl, Halogen, Hydroxy, Niederalkoxy oder Niederalkylthio stehen, oder
R₇ und R₈ zusammen einen Benzoring bilden, und
B 5-Tetrazolyl oder eine Gruppe -C(=O)-Y bedeutet, wobei
Y Hydroxy, Niederalkoxy, Amino, Niederalkylamino oder Diniederalkylamino bedeutet;
mit der Massgabe, dass A von einer Gruppe -C(R₄R₅)- verschieden ist, wenn B eine Gruppe -C(=O)-Y bedeutet; und Salze davon.

3. Verbindungen der Formel I gemäss Anspruch 1, worin
R₁ und R₂ unabhängig voneinander Wasserstoff, Niederalkyl, Trifluormethyl, Halogen, Niederalkoxy, Niederalkylthio oder Cyano bedeuten,
X für für eine Gruppe -CH₂-O- (wobei CH₂ mit dem Chinolinrest und O mit der Gruppe Ar verknüpft ist) oder eine Gruppe -CH=CH- steht,
Ar für 1,3-Phenylen steht,
R₃ Wasserstoff oder Niederalkyl bedeutet,
A für eine Gruppe -C(R₄R₅)- oder 1,2-Phenylen steht, wobei R₄ und R₅ unabhängig voneinander C₁-C₃-Alkyl bedeuten,
B 5-Tetrazolyl oder eine Gruppe -C(=O)-Y bedeutet, wobei Y Hydroxy, Niederalkoxy, Amino, Niederalkylamino oder Diniederalkylamino bedeutet;
mit der Massgabe, dass A von einer Gruppe -C(R₄R₅)- verschieden ist, wenn B eine Gruppe -C(=O)-Y bedeutet; und Salze davon.

4. Verbindungen der Formel I gemäss Anspruch 1, worin
R₁ und R₂ unabhängig voneinander Wasserstoff oder Halogen bedeuten,
X für für eine Gruppe -CH₂-O- (wobei CH₂ mit dem Chinolinrest und O mit der Gruppe Ar verknüpft ist) oder eine Gruppe -CH=CH- steht,
Ar für 1,3-Phenylen steht,
R₃ Wasserstoff bedeutet,
A für eine Gruppe -C(C₂H₅)₂- oder 1,2-Phenylen steht, und
B 5-Tetrazolyl oder -COOH bedeutet, wobei
mit der Massgabe, dass A von einer Gruppe -C(C₂H₅)₂- verschieden ist, wenn B eine Gruppe -COOH bedeutet; und pharmazeutisch verwendbare Salze davon.

5. 5-{3-[3-(7-Fluor-2-chinolinylmethoxy)phenylamino]-1,1-diethyl-3-oxopropyl}-1H-tetrazol gemäss Anspruch 1, oder ein pharmazeutisch anwendbares Salz davon.

6. 5-{3-[3-(7-Fluor-2-chinolinylvinyl)phenylamino]-1,1-diethyl-3-oxopropyl}-1H-tetrazol gemäss Anspruch 1, oder ein pharmazeutisch anwendbares Salz davon.

7. 2-[3-(7-Fluor-2-chinolinylmethoxy)phenylaminocarbonylmethyl]-benzoesäure gemäss Anspruch 1, oder ein pharmazeutisch anwendbares Salz davon.

8. 5-{2-[3-(7-Fluor-2-chinolinylmethoxy)phenylaminocarbonylmethyl]-phenyl}-1H-tetrazol gemäss Anspruch 1, oder ein pharmazeutisch anwendbares Salz davon.

9. Pharmazeutische Präparate enthaltend eine Verbindung gemäss einem der Ansprüche 1 bis 8 und mindestens ein pharmazeutisch verwendbares Trägermaterial.

10. Eine Verbindung gemäss einem der Ansprüche 1 bis 8 zur Anwendung in einem Verfahren zur therapeutischen Behandlung des tierischen oder menschlichen Körpers.

11. Eine Verbindung gemäss einem der Ansprüche 1 bis 8 zur Verwendung bei der Behandlung von Krankheiten, die auf eine Hemmung von Leukotrienen ansprechen.

12. Verwendung einer Verbindung gemäss einem der Ansprüche 1 bis 8 zur Herstellung pharmazeutischer Präparate.

13. Verwendung einer Verbindung gemäss einem der Ansprüche 1 bis 8 zur Herstellung pharmazeutischer Präparate für die Behandlung von allergischen und entzündlichen Prozessen.

14. Verfahren zur Herstellung einer Verbindung der Formel I gemäss Anspruch 1, dadurch gekennzeichnet, dass man
(a) eine Verbindung der Formel II, 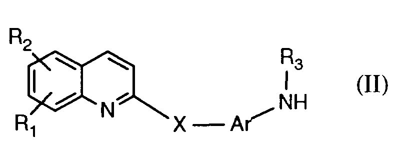 worin R₁,R₂,R₃, X und Ar wie unter Formel I definiert sind, mit einer Verbindung der Formel III, 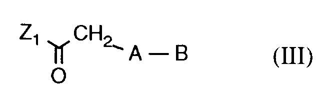 worin die Gruppe -COZ₁ Carboxy oder ein reaktionsfähiges Carboxyderivat bedeutet und A und B wie unter Formel I definiert sind, umsetzt, oder
(b) zur Herstellung von Verbindungen der Formel I, worin X für eine Gruppe -CH₂-O-steht, eine Verbindung der Formel IV, 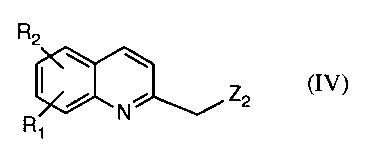 worin Z₂ eine nukleofuge Abgangsgruppe bedeutet und R₁ und R₂ wie unter Formel I definiert sind, mit einer Verbindung der Formel V, 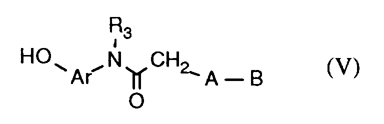 worin R₃, A, B und Ar wie unter Formel I definiert sind, oder einem Salz davon, umsetzt, oder
(c) zur Herstellung von Verbindungen der Formel I, worin B 5-Tetrazolyl bedeutet, eine Verbindung der Formel VII, 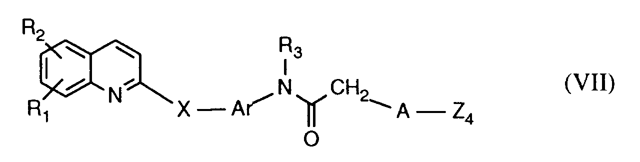 worin Z₄ Carboxy oder ein reaktionsfähiges Carboxyderivat, insbesondere Cyano, bedeutet, mit einem ringbildenden Agens, z.B. einem Azid, umsetzt;
und, wenn erwünscht, eine erhaltene Verbindung der Formel I in eine andere Verbindung der Formel I umwandelt, und/oder, wenn erwünscht, ein erhaltenes Salz in die freie Verbindung oder in ein anderes Salz umwandelt, und/oder, wenn erwünscht, eine erhaltene freie Verbindung der Formel I mit salzbildenden Eigenschaften in ein Salz umwandelt.
